# FASCICULE DE BREVET EUROPEEN

(11) **EP 1 465 653 B1**
(45) Date de publication et mention de la délivrance du brevet: **10.08.2011**
(21) Numéro de dépôt: 03712209.0
(22) Date de dépôt: 06.01.2003
(51) Int. Cl.: A61K 38/19, A61K 38/18, A61P 25/00

(54) **APPLICATION THERAPEUTIQUE DU G-CSF**
THERAPEUTISCHE ANWENDUNG VON G-CSF
THERAPEUTIC APPLICATION OF G-CSF

(30) Priorité: 18.01.2002 FR 0200610
(43) Date de publication de la demande: 13.10.2004
(60) Demande divisionnaire: 10180416.9
(73) Titulaire: Pourquier, Didier, 34070 Montpellier (FR); Moukoko, Didier, 34980 Saint Gely du Fesc (FR)
(72) Inventeur: Pourquier, Didier, 34070 Montpellier (FR); Moukoko, Didier, 34980 Saint Gely du Fesc (FR)
(74) Mandataire: Ahner, Francis
(86) Numéro de dépôt international: PCT/FR2003/000013
(87) Numéro de publication internationale: WO 2003/061685

(56) Documents cités:
- EP-A1- 1 374 898
- WO-A-99/17798
- DE-A- 10 033 219
- W.R. SCHÄBITZ ET AL.: "Recombinant granulocyte-colony stimulating factor (rG-CSF) is neuroprotective following focal transient cerebral ischemia and excitotoxicity." SOCIETY FOR NEUROSCIENCE ABSTRACTS; 31ST ANNUAL MEETING, SAN DIEGO, CA, NOVEMBER 10-15, 2001, vol. 27, no. part 2, 2001, page 2027 XP008009334
- D. ORLIC ET AL.: "Mobilized bone marrow cells repair the infarcted heart, improving function and survival." PROCEEDINGS OF THE NATIONAL ACADEMY OF SCIENCES OF USA, vol. 98, no. 18, 28 août 2001 (2001-08-28), pages 10344-10349, XP002216659 WASHINGTON, US cité dans la demande
- É. MEZEY ET AL.: "Turning blood into brain: Cells bearing neuronal antigens generated in vivo from bone marrow." SCIENCE, vol. 290, 1 décembre 2000 (2000-12-01), pages 1779-1782, XP002216660 LANCASTER, PA, US cité dans la demande
- T. TAKAHASHI ET AL.: "Ischemia-and cytokine-induced mobilization of bone marrow-derived endothelial progenitor cells for neovascularization." NATURE MEDICINE, vol. 5, no. 4, avril 1999 (1999-04), pages 434-438, XP002216661 NEW YORK, N.Y., US cité dans la demande

## Description

L'invention concerne une nouvelle application thérapeutique du G-CSF et du SCF, et plus particulièrement leur utilisation pour la préparation d'un médicament utile en médecine humaine ou vétérinaire.

Le G-CSF (abréviation de "Granulocyte Colony-Stimulating Factor", c'est à dire "Facteur de Stimulation des Colonies de Granulocytes"), le GM-CSF (abréviation de "Granulocyte-Macrophage Colony-Stimulating Factor", c'est à dire "Facteur de Stimulation des Colonies de Granulocytes et de Macrophages") et le SCF (abréviation de "Stem Cell Factor", c'est à dire "Facteur des Cellule Souches") sont des facteurs de croissance. Ils correspondent à trois des classes de cytokines appelées facteurs de croissance hématopoïétique ou CSF (Colony-Stimulating Factors). Les CSF forment une famille de glycoprotéines ayant des fonctions capitales dans la formation des cellules sanguines.

Le G-CSF stimule la production des cellules hématopoïétiques et de manière prédominante la production de polynucléaires. Le G-CSF est une glycoprotéine et le produit de l'expression d'un gène situé sur le chromosome 17. Il est produit par différentes cellules, telles que les fibroblastes, macrophages, cellules endothéliales, cellules épithéliales. Le G-CSF est détectable dans le sang, il peut être purifié à partir de surnageants de cultures de cellules tumorales humaines.

Le génie biologique permet aussi de produire un allotype humain de G-CSF, le G-CSF recombinant humain (rBuG-CSF).

En France deux médicaments appartenant à la classe du G-CSF sont disponibles : le Neupogen^{®} (r-metHuG-CSF, Filgrastime, commercialisé par Amgen/Produits Roche), et le Granocyte^{®} (rHuG-CSF, Lénograstime, commercialisé par les laboratoires Aventis/Chugai). Ces deux médicaments sont utilisés par voie d'injection sous-cutanée ou par perfusion intraveineuse, pour une administration systémique, à des doses généralement comprises entre 5 et 10 µg par kilogramme de poids corporel et par jour.

Les indications actuelles du G-CSF en thérapeutique humaine sont le traitement des neutropénies chroniques sévères, la réduction des neutropénies induites par les traitements chimiothérapiques anticancéreux myélotoxiques, la réduction des neutropénies induites par les thérapeutiques myélosuppressives (chimiothérapie ou radiothérapie) suivies de greffe de moelle dans le traitement des cancers ou des leucémies, la mobilisation des cellules souches hématopoïétiques pour constitution d'un greffon en vue d'une greffe de moelle (autogreffe ou allogreffe).

Il est à souligner que, dans cette dernière utilisation, l'objectif du traitement est de faire sortir de la moelle osseuse les cellules souches hématopoïétiques et de les faire passer dans le sang circulant. Ces cellules souches sont alors recueillies par cytaphérèses successives et vont constituer le greffon. Il s'agit donc de mobiliser au maximum vers le sang circulant ce contingent de cellules souches hématopoïétiques qui, à l'état normal, se trouvent en quantité très réduite dans le sang circulant, ce qui ne permet pas de constituer un greffon de qualité suffisante (ceci est résumé dans le terme "mobilisation" de la moelle osseuse).

Cette technique de recueil du greffon par cytaphérèse après mobilisation des cellules souches est venue remplacer avantageusement le recueil direct de la moelle osseuse par cytoponction qui nécessite une anesthésie du patient et de multiples ponctions de moelle. Le greffon ainsi constitué est ensuite transfusé au patient, dont la propre moelle a été détruite par la chimiothérapie ou la radiothérapie ; il constitue alors la nouvelle source de production des cellules sanguines.

Le G-CSF est utilisé de manière quotidienne pour ces différentes indications. Son innocuité largement reconnue permet de l'utiliser aussi chez des personnes en bonne santé pour constituer des greffons de moelle dans le cadre des allo-greffes.

Le SCF (abréviation de "Stem Cell Factor", c'est à dire "Facteur de Cellules Souches") est lui aussi un facteur de croissance. Il agit en particulier comme facteur de croissance sur les progéniteurs hématopoïétiques, et peut mobiliser les cellules souches de la moelle vers le sang. Il agit aussi sur la différenciation et le fonctionnement des mastocytes. Il agit par le biais d'un récepteur spécifique de la famille des tyrosines kinases de type III(c-KIT). Le gène codant la production du SCF est situé sur le chromosome 12. En France le SCF n'est pas d'usage clinique quotidien, il est disponible pour des essais thérapeutiques sous forme de recombinant humain (Ancestim, Recombinant-methionyl Human Stem Cell Factor (R-metHuSCF){dénomination anglaise} sous la dénomination de Stemgen^{®} {dénomination anglaise}, produit de la société Amgen).

Il est à noter que les recombinants humains du G-CSF sont efficaces sur d'autres espèces animales (Gratwohl et col : Transplantation of G-CSF mobilized allogeneic peripheral blood stem cells in rabbits. Bone Marrow Transplant 1995 ;16(1):63-68) ; Nohynek GJ et col: Comparison of potency of glycosylated and non glycosylated recombinant human granulocyte colony-stimulating factors in neutropenic and non neutropenic CD rats (Cancer Chemother Pharmaco1.1997;39:259-266).

Il est à noter que de nouvelles classes de molécules peuvent augmenter le pouvoir mobilisateur des facteurs de croissance sur la moelle osseuse (Kronenwett R et col: The role of cytokines and adhesion molecules for mobilisation of peripheral blood stem cells. Stem Cells 2000;18:320-330; Pless M et col:. Synergy of growth factors during mobilization of peripheral blood precursors cells with recombinant Flt3-ligand and granulocyte colony-stimulating factor in rabbits. Exp Hematol 1999;27:155-161 ; Kikuta T et col: Mobilization of hematopoietic primitive and commited progenitor cells into blood in mice by anti-vascular adhesion molecule-1 antibody alone or in combination with granulocyte colony-stimulating factor. Exp Hematol.2000;28:311-317 ; Sweeney EA et col:Increase in circulating SDF-1 after treatment with sulfated glycans. The role of SDF-1 in mobilization. Ann N Y Acad Sci 2001;938:48-52 ; Papayannopoulou R et col: Synergistic mobilization of hematopoietic progenitor cells using concurent betal and beta2 integrin blockade or beta2-deficient mice. Blood 2001;97:1282-1288 ; Christ 0 et col: Combining G-CSF with a blockade of adhésion strongly improves the reconstitutivé capacity of mobilized hematopoietic progenitor cells. (Exp Hematol 2001;29:380-390).

Cependant le G-CSF lui même possède un pouvoir mobilisateur indirect de la moelle en plus de son effet prolifératif sur la lignée hématopoïétique (Levesque JP et col: Vascular cell adhésion molecule-1(CD106) is cleaved by neutrophil proteases in the bone marrow following hematopoietic progenitor cell mobilization by granulocyte colony-stimulating factor. Blood 2001;98:1289-1297).

Il convient de rappeler que la moelle osseuse, répartie dans les différents os de l'organisme, est le lieu de production des cellules sanguines matures (érythrocytes, plaquettes, polynucléaires, monocytes, lymphocytes). La pro duction de ces cellules sanguines s'effectue à partir de la multiplication et de la différenciation d'une population de cellules souches (Cellule Souche Hématopoïétique, en abréviation "CSH"). Ces dernières représentent quantitativement une fraction très réduite de la population cellulaire de la moelle osseuse. Elles sont classiquement caractérisées par l'expression d'un marqueur cellulaire appelé CD34 (Cluster de Différenciation 34).

Cependant il a été décrit un autre type de cellules souches de la moelle osseuse. Cette population de cellules souches constitue, elle aussi, à l'état normal un contingent très réduit des éléments cellulaires de la moelle osseuse, mais présente la capacité de se différencier dans de multiples directions de nature conjonctive (os, cartilage, fibrotendon, muscle, graisse) (Pittenger et col: Multilineage potential of human mesenchymal stem cells. Science 1999 ; 284 : 143-147; Dennis et col: A quadripotential mesenchymal progenitor cell isolated from the marrow of an adult mouse. J Bone Miner Res 1999 ; 14(5): 700-709 ; Seshi et col: Human bone marrow stromal cell: coexpression of markers specific for multiple mesenchymal stem lineage. Blood Cell Mol Dis 2000(3): 234-246), mais aussi de nature nerveuse (Woodbury D et col: Adult rat and human bone marrow stromal cells differentiate into neurons. J Neurosci Res 2000;61:364-370 ; Sanchez-Ramos J et col: Adult bone marrow stromal cells differentiate into neural cells in vitro. Exp Neurol.2000;164:247-256 ; Mezey E et col: Turning blood into brain: cells bearing neuronal antigens generated in vivo from bone marrow. Sience 2000;290:1672-1674 ; Azizi SA et col: Engraftment and migration of human bone marrow stromal cells implanted in brains of albino rats - similarities to astrocytes graft. Proc Nat Acad Sci.1998;95:3908-3913 ; Kopen GC et col: Marrow stromal cells migrate throughout forebrain and cerebellum, and they differentiate into astrocytes aster injection into neonatal mouse brains. Proc Nat Acad Sci.1999;96;10171-10716 ; Brazelton TR et col: From marrow to brain: expression of neuronal phenotypes in adult mice. Science 2000;290:1775-1779), endothéliale (Shi Q et col: Evidence for circulating bone marrow-derived endothelial cells. Blood 1998;92:362-367 ; Asahara T et col: Bone marrow origin of endothelial progenitor cells responsible for post natal vasculogenesis in physiological and pathological neovascularization. Circ Res 1999;85:221-228) ou encore hépatique (Lagasse E et col: Purified hematopoietic stem cells can differentiate into hepatocytes in vivo. Nature Medecine.2000;6:1229-1234).

La moelle osseuse en tant que source particulièrement puissante de cellules souches pluripotentes est clairement indiquée par le récent travail de Krause et collaborateurs (Krause D et col: Multi-organ, multi-lineage engraftment by a single bone marrow-derived stem cell. Cell 2001; 105:369-377), avec dans cette dernière étude un réensemencement de la totalité du tissu hématopoïétique à partir d'une unique cellule greffée, et des voies de différenciations in vivo très nombreuses avec des cellules "filles" retrouvées dans de multiples organes (foie, tube digestif, poumon, peau). Ces cellules souches sont appelées cellules souches pluripotentes (en abréviation "CSP") ou aussi cellules souches adultes par opposition aux cellules souches provenant d'embryons.

Il est à noter que la moelle osseuse n'est pas l'unique source de cellules souches. Certaines zones du cerveau peuvent donner des cellules souches pluripotentes (Bjornson CRR et col: Turning brain into blood:a hematopoietic fate adopted by adult neural stem cells in vivo. Science 1998;283:534-537 ; Rietze RL et col: Purification of a pluripotent neural stem cell from the adult mouse brain. Nature 2001;412:736-739), de même que le muscle (Jackson KJ et col: Hematopoietic potential of stem cells isolated from murine skeletal muscle. Proc Natl Acad Sci USA.1999;96:14482-14486), ou la peau (Fu X et col: Dedifferentiation of epidermal cells to stem cells in vivo; Lancet 2001;358:1067-1068 ; Toma JG et col:Isolation of multipotent adult stem cells from the dermis of mammalian skin. Nat Cell Biol 2001;3:778-784).

Cependant la moelle osseuse est à ce jour la source la mieux connue de cellules souches, et aussi la source la plus aisément "manipulable" pharmacologiquement, en particulier à cause de la grande expérience clinique acquise dans le domaine de l'hématologie et de la cancérologie.

Cette population de cellules souches est l'objet actuellement d'une activité de recherche intense, en particulier dans les domaines de la réparation osseuse, de la cardiologie, de la neurologie, de l'hématologie, ceci dans le cadre des domaines émergents de la médecine que sont l'ingénierie tissulaire et la thérapie cellulaire. Cependant, ces cellules souches constituant au départ une quantité de cellules particulièrement faible, les techniques utilisées font le plus souvent appel à un passage in vitro. Pour résumer, on recueille la moelle osseuse, puis in vitro on sépare le contingent des cellules souches du reste des cellules de la moelle, et ces cellules sont ensuite cultivées et multipliées toujours in vitro. Eventuellement elles sont aussi poussées artificiellement dans le sens d'une différenciation spécifique. Par la suite elles sont réinjectées ou réimplantées dans un site anatomique où elles contribuent à la reconstitution d'un tissu délabré, qui peut être un os (Bruder et col: Bone régénération by implantation of purified culture-expended human mesenchymal stem cells. J Ortho Res 1998 ; 16(2) : 155-162) ou bien un tendon (Awad HA et col: Autologous mesenchymal stem cell-mediated repair of tendon. Tissue Eng 1999; 5:267-277 ; Butler DL et col: Perspectives on cell and collagen composites for tendon repair. Clin Orthop 1999; 367 Suppl : S 324-332), du muscle cardiaque (Jackson KA et col: Régénération of ischémic cardiac muscle and vascular endothelium by adult stem cells. J Clin Invest 2001;107:1395-1402 ; Orlic D et col: Bone marrow cells regenerate infarcted myocardium. Nature 2001;410:702-705). Ces techniques nécessitent cependant une collaboration avec des laboratoires très spécialisés, ce qui limite leur usage clinique quotidien.

Les cellules souches de la moelle sont aussi d'un grand intérêt dans le cadre de la thérapie génique où elles peuvent servir de support au transfert de gènes. De multiples domaines de la pathologie sont concernés (Schwarz EJ et col:Multipotential marrow stroma cells transducted to produce L-DOPA: engraftment in rat model of Parkinson disease. Hum Gene Ther 1999;10:2539-2545 ; Ding L et col:Bone marrow stromal cells as a vehicle for gene transfer. Gene Ther 1999;6:1611-1616).

Bien qu'ayant initialement prêté à discussions (Purton LE et col: Monocytes are the likely candidate "stromal" cell in G-CSF-mobilized péripheral blood. Bone Marrow Transplant.1998;21:1075-1076), la présence d'un contingent circulant de cellules souches pluripotentes (Cellules Souches Pluripotentes Circulantes en abréviation "CSPC") est actuellement de mieux en mieux documenté (Huss R et col: Evidence of peripheral blood-derived, plastic-adherent CD34(-/low) hematopoietic stem cell clones with mesenchymal stem cell characteristics. Stem Cell 2000 ; 18(4) : 252-260 ; Zvaifler NJ et col : Mesenchymal precursor cells in blood of normal individuals; Arthritis Res 2000;2:477-488 ; Lange et col : Hematopoietic reconstruction of syngenic mice with a peripheral blood-derived, monoclonal CD34-, Sca-1+, Thyl(low), c-kit+ stem cell ligne. J Hematother Stem Cell Res. 1999 ; 8(4): 335-342 ; Kuznetsov SA et col:Circulating skelatal stem cells: J Cell Biol 2001;153:1133-1140). La voie de différenciation endothéliale de ces cellules est aussi explorée (Rafii S: Circulating endothelial precursors:mystery, reality and promise. J Clin Invest 2000;105:17-19, Boyer M et col: Isolation of endothelial cells and their progenitor cells from human peripheral blood. J Vasc Surg 2000;31:181-189). La mobilisation de cellules souches depuis la moelle osseuse vers le sang par l'ischémie des tissus (Takahashi T et col: Ischemia-and cytokine-induced mobilization of bone marrow-derived endothelial progenitor cells for neovascularization Nature Medecine 1999; 5:434-438), les traumatismes vasculaires ou les brûlures est aussi documenté (Gill M et col: Vascular trauma induces rapid but transient mobilization of VEGFR2(+)AC133(+) endothelial precursor cells. Cir Res 2001;88:167-174). Le VEGF (Vascular Endothelial Growth Factor) serait un des médiateurs impliqués dans le processus de mobilisation (Asahara T et col: VEGF contributes to postnatal neovascularisation by mobilizing bone marrow-derived endothelial progenitor cells. EMBO J 1999;18:1364-1372 ; Moore MA et col: Mobilization of endothelial and hematopoietic stem and progenitor cells by adenovector-mediated élévation of sérum levels of SDF-1,VEGF, and angiopoietin-1. Ann N Y Acad Sci 2001;938:36-45). Ces derniers travaux pourraient indiquer qu'un processus de mobilisation des cellules souches de la moelle osseuse est un mécanisme physiopathologique déjà utilisé par l'organisme dans certaines situations pathologiques.

De même que pour les cellules souches de la moelle, les CSPC sont un support au transfert de gènes (Bodine DM et col: Efficient retrovirus transduction of mouse pluripotent hematopoietic stem cell mobilized into the peripheral blood by treatment by granulocyte colony-stimulating factor and stem cell factor. Blood 1994;84:1482-1491).

Des travaux récents sont aussi venus suggérer un mécanisme de recrutement des CSPC dans les processus de cicatrisation fibroconjonctive sur des modèles animaux in vivo (Abe R et col: peripheral blood fibrocytes: Différenciation patways and migration to wound sites. The J Immunol 2001;166:7556-7562 ; Bucala R et col: Circulating fibrocytes define a new leukocyte subpopulation that médiates tissue repair. Mol Med.1994;1:71-81). Les CSPC sont aussi soupçonnées de participer à la pathogénèse de processus de fibrose pathologique (sclérodermie) (Chesney J et col: Peripheral blood fibrocytes:mesenchymal precursor cells and the pathogenesis of fibrosis. Curr Rheumatol Rep.200;2:501-505). L'implication de ces cellules souches circulantes était cependant soupçonnée depuis longtemps dans certaines pathologies spécifiques (Labat ML et col: Monocytic origin of fibroblasts:spontaneous transformation of blood monocytes into neofibroblastic structures in osteomyeloslerosis and Engelmann's disease. Biomed Pharmacother.1991;45:289-299 ; Labat ML et col: Possible monocytic origin of chondrosarcoma: in vitro transdifferenciation of blood monocytes-like cells from a patient with chondrosarcoma into chondrocyte-like cell. Biomed Pharmacother 1997 ; 51:79-93). Des mécanisme immunologiques originaux viendraient aussi contrôler la prolifération et la différenciation de ces CSPC (Labat M L et col: Regulation by phagic T-lymphocytes of a (pluripotent?)organ stem cell présent in adult human blood. A beneficial exception to self-tolérance. Biomed Pharmacother 2001;55:79-90).

A rappeler aussi qu'au cours des processus de réparation tissulaire, il existe d'une manière générale une contribution de cellules souches locales. Les cellules basales de l'épiderme contribuent ainsi à la réparation de la peau, les cellules satellites du muscle contribuent de même à la réparation musculaire, les éléments cellulaires de la couche basale du périoste contribuent classiquement à la reconstruction osseuse. Certains tissus comme l'os en cas de fracture ou la peau en cas de brûlure superficielle sont capables d'une reconstitution "ad integrum".

D'autres tissus, comme le coeur et le cerveau, étaient jusqu'à maintenant réputés incapables de se régénérer après leur destruction (infarctus du myocarde ou accident vasculaire cérébral). Cependant des travaux récents ont montré en fait une certaine capacité du tissu cardiaque à se régénérer à partir des cellules de la bordure des zones lésées (Beltrami AP et col: Evidence that human cardiac myocytes divide after myocardial infarction. New Engl J Med 2001;344:1750-1757). L'activation de cellules souches déjà présentes au niveau du cerveau est aussi discutée (Kondo T et col: Oligodendrocytes precursor cells reprogrammed to become multipotential CNS stem cells: Science 2000;289:-1754-1757), remettant en cause des concepts anciens, en particulier le caractère irréversible de la différenciation cellulaire. La place des CSPC dans ces processus de régénération est encore discutée, mais les travaux de thérapie cellulaire récents indiquent des possibilités importantes pour le coeur (Jackson KA et col: Régénération of ischemic cardiac muscle and vascular endothelium by adult stem cells. J Clin Invest 2001;107:1395-1402 ; Orlic D et col: Bone marrow cells regenerate infarcted myocardium. Nature 2001;410:702-705 ; Kocher AA et col: Neovascularisation of ischemic myocardium by human bone-marrow-derived angioblast prevents cardiomyocyte apoptosis, reduces remodeling and improves cardiac function. Nature Médecine. 2001;7:430-436). Il est à remarquer que dans le cas des travaux de Kocher AA et col, le "greffon" de cellules souches se fait non pas à partir d'un prélèvement direct de moelle, mais à partir des cellules souches mobilisées depuis la moelle osseuse vers le sang, suggérant le même potentiel biologique pour les CSPC que pour les CSP de la moelle osseuse. Le travail de Jackson KA et col est aussi d'un grand intérêt puisque après irradiation et greffe de moelle de cellules souches marquées, ces travaux indiquent aussi une migration des cellules souches marquées vers le site intracardiaque de l'infarctus du myocarde. On retrouve ici la notion de recrutement spécifique des cellules souches circulantes dans l'espace tissulaire en cours de reconstruction.

Dans le domaine de la pathologie cérébrale la délivrance locale de cellules souches semble apporter un bénéfice thérapeutique (Eglitis MA et col: Targeting of bone marrow-derived astrocytes to the ischemic brain. Neuroreport 1999;10:1289-1292 ; Lu D et col: Intraarterial, administration of marrow stromal cells in a rat model of traumatic brain injury. J Neurotrauma 2001;18:813-819 ; Chen J et col: Therapeutic benefit of intracerebral transplantation of bone marrow stromal cells after cerbral ischemia in rats. J Neurol Sci.2001;189:49-57; Mahmood A et col: Intracranial bone marrow transplantation after traumatic brain injury improving functional outcome in adult rats. J Neurosurg 2001;94:589-595).

Au total il ressort de l'ensemble de ces observations que la moelle osseuse constitue une réserve majeure de cellules souches pluripotentes. Ces cellules souches sont retrouvées dans le sang circulant, le nombre de CSPC peut être augmenté par l'utilisation de facteurs de croissance tels que le GCSF, le GM-CSF ou le SCF par mobilisation de ces cellules souches de la moelle osseuse vers le sang. Ce processus de mobilisation des cellules souches de la moelle vers le sang pourrait exister à l'état naturel comme mécanisme de réponse à des situations de souffrance ou d'agression tissulaire. Un mécanisme de recrutement à l'état naturel des CSPC est aussi discuté en particulier dans les processus de réparation fibrocicatriciel, mais a aussi été suggéré sur des modèles expérimentaux de souffrance tissulaire et des modèles pathologiques spécifiques. Les Demandeurs ont largement exposé dans le cadre de la pathologie ostéo-articulaire l'intérêt de la mobilisation des cellules souche de la moelle osseuse par le G-CSF et l'existence d'un recrutement de ces cellules dans les espaces tissulaires de reconstruction de l'os.

Différents travaux des Demandeurs ont déjà indiqué que dans le domaine de la pathologie ostéoarticulaire un mécanisme de recrutement local des CSPC au cours des fractures osseuses était mis en oeuvre en particulier par le biais de l'activation du périoste. Ces cellules souches, en sortant des vaisseaux, sont mises à la disposition du processus de reconstruction tissulaire local comme des "briques" contribuant à la construction d'un édifice et viennent compléter l'apport direct des cellules souches locales. En terme quantitatif, l'importance du recrutement local de CSPC est cependant dépendante du nombre de cellules souches présentes dans le courant sanguin au niveau des vaisseaux situés dans la zone de reconstruction tissulaire. Comme déjà mentionné, cette population de cellules souches est, chez le sujet en bonne santé, quantitativement très réduite dans la moelle osseuse et le sang circulant. Cependant, le G-CSF, le GM-CSF et le SCF possèdent un puissant pouvoir prolifératif et de mobilisation de la moelle osseuse. Bien que la lignée hématopoïétique soit surtout concernée par ce pouvoir prolifératif, le contingent de cellules souches pluripotentes est aussi concerné ; ces différents facteurs de croissance en augmentant le nombre de CSPC dans le sang circulant peuvent mettre à la disposition du processus de reconstruction tissulaire un nombre de cellules souches supérieur à celui que la nature aurait pu fournir à l'état naturel. A partir de la même hypothèse physiopathologique l'utilité du G-CSF comme traitement adjuvant au processus de réparation osseuse et cartilagineuse était démontré dans les travaux des Demandeurs. Les travaux récents menés dans le domaine de la pathologie cardiaque semblent conforter cette hypothèse avec un bénéfice thérapeutique du G-CSF combiné au SCF pour le traitement de l'infarctus du myocarde en réduisant la surface finale de l'infarctus et en préservant la fonction cardiaque. (Orlic D et col: Mobilized bone marrow cells repair the infarcted heart, improving function and survival Proc Natl Acad Sci USA 2001 ;98 :10344-10349).

Dans ce contexte, les Demandeurs ont testé in vivo l'influence d'un traitement par facteurs de croissance sur un accident vasculaire cérébral. Sur la base des données précédentes, les Demandeurs ont étudié l'influence de la stimulation de la moelle osseuse par le G-CSF, le GM-CSF et le SCF sur le processus de réparation du tissu nerveux.

Les Demandeurs ont trouvé, de manière surprenante, une nouvelle application thérapeutique du G-CSF et du SCF tel que définis plus haut. Il a été découvert, à la suite de ces travaux, que le G-CSF et le SCF pouvaient être utilisés dans la préparation d'un médicament pour le traitement adjuvant au processus de reconstruction des tissus nerveux chez un être vivant, le médicament étant administré par voie générale.

Conformément à l'invention on peut utiliser l'un au moins de ces deux facteurs, c'est-à-dire soit un seul facteur, soit les deux facteurs .

Il est à rappeler que l'accident vasculaire cérébral ischémique est la conséquence de l'obstruction d'un vaisseau sanguin irriguant le tissu cérébral. La destruction du tissu nerveux privé d'oxygène dans le territoire anatomique concerné entraîne un déficit fonctionnel de type moteur, sensitif ou cognitif ; il existe aussi des accidents vasculaires de type hémorragique (par exemple à la suite d'une poussée d'hypertension artérielle ou par rupture d'anévrysme). Dans ce cas le parenchyme cérébral est détruit par la suffusion hémorragique, les traumatismes crâniens et les traumatismes de la moelle épinière entraînent des destructions directes du tissu nerveux.

On décrira maintenant un exemple expérimental.

Deux groupes d'animaux ont été constitués:
Un premier groupe (A) (groupe témoin) comportait 20 rats adultes jeunes, et on réalisait sur ce groupe un accident vasculaire cérébral ischémique par ligature de l'artère cérébrale moyenne durant deux heures.
Un deuxième groupe (B) comportait un nombre identique d'animaux de même âge et de même poids et on réalisait le même type d'accident vasculaire. Cependant, immédiatement après l'intervention et les quatre jours suivant l'intervention on procédait à une injection de rmetHuG-CSF (Neupogen^{®} {Filgrastime}) par voie sous cutanée à la dose de 10 µg (1MU) par kilogramme de poids corporel.

Afin de travailler véritablement "en aveugle" et dans le but de ne pas influencer la qualité des gestes- chirurgicaux de ligature artérielle et de retrait de ligature réalisés dans chaque groupe, le chirurgien ignorait si l'animal opéré appartenait au groupe A ou au groupe B.

Les deux groupes d'animaux étaient ensuite laissés sans autre traitement, hormis l'apport en eau et nourriture nécessaire aux besoins vitaux. Quinze jours suivant la date de l'intervention les animaux subissaient des tests fonctionnels neurologiques (Rotarod test, Score de sévérité neurologique modifié). Les résultats indiquaient une réduction des déficits fonctionnels dans le groupe B par rapport au groupe témoin.

L'invention concerne donc plus particulièrement l'utilisation de l'un au moins des facteurs choisis parmi le G-CSF, et le SCF dans la préparation d'un médicament pour le traitement adjuvant dans des processus de reconstruction des tissus-nerveux, le médicament étant destiné à une administration par voie générale. Ces facteurs trouvent ainsi une application nouvelle et intéressante dans les thérapeutiques chirurgicales et/ou médicales utilisées dans le cadre de la patrologie du système nerveux.

Conformément à l'invention, le G-CSF, le SCF peuvent être utilisés comme médicament dans le traitement des accidents vasculaires cérébraux ischémiques ou hémorragiques, des traumatismes cérébraux, des accidents vasculaires hémorragiques ou ischémiques de la moelle épinière, des traumatismes de la moelle épinière. Dans ces différentes applications le G-CSF, le SCF sont destinés à l'administration par voie générale.

Il entre également dans le cadre de l'invention d'utiliser le G-CSF, ou le SCF comme médicament destiné à l'administration par voie générale et de le combiner à au moins un autre facteur destiné à l'administration locale ou par voie générale. L'expression "administration locale" signifie que l'administration du principe thérapeutique s'effectue sur le site anatomique où l'on souhaite reconstruire le tissu nerveux.

Cet autre facteur est avantageusement choisi parmi l'un au moins des facteurs suivants : BMP (Bone Morphogenetic Protein), FGF (fibroblast growth factor), EGF (epidermal growth factor), IGF (insuline-like growth factor), Insuline, KGF (keratinocyte growth factor), TGF (transforming growth factor), Interféron, Interleukine, VEGF (vascular endothélial growth factor), TNF (tumor necrosing factor), GDNF (glial cell ligne-derived neurotrophic factor), NGF (neurotrophin nerve growth factor), HGF (hepatocyte growth factor), Erythropoïetine, PDGF (platelet-derived growth factor), Héparane Sulfate, Prostaglandines, Ostéoglycine (osteoinductive factor), BCDF (B cell differentiation factor), GDF-5 (growth and differentiation factor-5), Hormone de Croissance ; M-CSF (macrophage colony stimulating factor) ; ou tout facteur de croissance connu d'origine humaine ou animale, d'extraction ou recombinant ; tout facteur biologique renforçant le pouvoir de mobilisation des cellules souches de la moelle osseuse vers le sang circulant.

Pour la mise en oeuvre de l'invention, le G-CSF, le SCF sont avantageusement un recombinant humain (pour la médecine humaine), ou la Filgrastime, la Lénograstime, l'Ancestim.

Le G-CSF et le SCF utilisés seront de préférence d'un allotype humain dans le cadre du traitement d'une personne humaine. Le dosage utilisé sera généralement de 0,1 à 1000 µg par kilogramme de poids corporel et par jour. De manière préférentielle on utilisera une dose de 5 à 10 µg par kilogramme de poids corporel et par jour.

Le principe de l'invention veut que le G-CSF, le SCF soient administrés par voie générale, ce qui signifie que le G-CSF, le SCF, entrent dans la circulation sanguine générale. Le mode de délivrance pour obtenir une administration par voie générale peut comprendre un mode par injection intravasculaire directe, un mode par injection sous-cutanée, par injection intramusculaire, par injection intra-articulaire, une délivrance par voie digestive, injection intrapéritonéale, délivrance transpulmonaire, transcutanée, transbuccale, transnasale, transrectale, transconjonctivale, intrarachidienne.

Le principe biologique de l'invention est de mobiliser les CSP de la moelle osseuse vers le sang circulant. Cette augmentation du nombre de cellules souches dans la circulation sanguine permet au processus biologique de reconstruction du tissu nerveux de recruter une quantité de CSPC supérieure à la quantité de CSPC que la nature aurait pu fournir à l'état physiologique.

L'invention sera maintenant décrite en référence à un exemple clinique.

Dans le cadre du traitement des accidents vasculaires cérébraux, on utilisera le traitement standard de réanimation médicale, de recherche et de traitement de la cause de l'accident vasculaire cérébral.

Le traitement adjuvant comportera une première dose de 10 µg (1 MU) par kilogramme de poids corporel de G-CSF (Neupogen^{®}, Filgrastime) qui sera injectée par voie sous-cutanée au patient dès son admission à l'hôpital. La même dose sera injectée de manière quotidienne pendant les quatre jours suivants.

Le même principe de traitement adjuvant pourra être appliqué aux traumatismes cérébraux et aux traumatismes de la moelle épinière.

Bien entendu l'exemple ci-dessus est donné seulement à titre illustratif et n'entend pas limiter la portée de l'invention.

## Revendications

1. Utilisation du G-CSF (Facteur de Stimulation des Colonies de Granulocytes) pour la préparation d'un médicament utile comme traitement adjuvant dans un processus de reconstruction des tissus nerveux chez un être vivant, le médicament étant destiné à l'administration par voie générale.

2. Utilisation selon la revendication 1, **caractérisée en ce que** le G-CSF est utilisé comme médicament dans le traitement des accidents vasculaires cérébraux ischémiques ou hémorragiques, des traumatismes cérébraux, des accidents vasculaires hémorragiques ou ischémiques de la moelle épinière, des traumatismes de la moelle épinière.

3. Utilisation selon l'une des revendications 1 et 2, **caractérisée en ce que** le G-CSF est combiné à au moins un autre facteur destiné à l'administration locale ou par voie générale.

4. Utilisation selon la revendication 3, **caractérisée en ce que** cet autre facteur est choisi parmi l'un au moins des facteurs suivants : BMP (bone morphogenetic protein), FGF (fibroblast growth factor), EGF (epidermal growth factor), IGF (insuline-like growth factor), Insuline, KGF (keratinocyte growth factor), TGF (transforming growth factor), Interféron, Interleukine, VEGF (vascular endothélial growth factor), TNF (tumor necrosing factor), GDNF (glial cell ligne-derived neurotrophic factor), NGF (neurotrophin nerve growth factor), HGF (hepatocyte growth factor), Erythropoïétine, PDGF (platelet-derived growth factor), Héparane Sulfate, Prostaglandines, Ostéoglycine (osteoinductive factor), BCDF (B cell differentiation factor), GDF-5 (growth and differentiation factor-5), Hormone de Croissance ; M-CSF (macrophage colony stimulating factor) ; tout facteur de croissance connu d'origine humaine ou animale, d'extraction ou recombinant ; tout facteur biologique renforçant le pouvoir de mobilisation des cellules souches de la moelle osseuse vers le sang circulant.

5. Utilisation selon l'une des revendications 1 à 4, **caractérisée en ce que** le G-CSF est un recombinant humain.

6. Utilisation selon l'une des revendications 1 à 4, **caractérisée en ce que** le G-CSF est la Filgrastime.

7. Utilisation selon l'une des revendications 1 à 4, **caractérisée en ce que** le G-CSF est la Lénograstime.

8. Utilisation selon l'une des revendications 1 à 7, **caractérisée en ce que** le médicament est utilisé avec un dosage en G-CSF de 0,1 à 1000 µg, de préférence de 5 à 10 µg, par kilogramme de poids corporel et par jour.

9. Utilisation selon l'une des revendications 1 à 7, **caractérisée en ce que** le médicament est utilisé en médecine humaine.

10. Utilisation selon l'une des revendications 1 à 7, **caractérisée en ce que** le médicament est utilisé en médecine vétérinaire.

## Claims

1. Use of G-CSF (Granulocyte-colony stimulating factor) for the preparation of a drug useful as an adjuvant treatment in the process of nervous tissue reconstruction in a living being, the drug being intended for administration by systemic route.

2. Use according to claim 1, **characterized in that** G-SCF is used as a drug in the treatment of ischemic or hemorrhagic cerebral vascular accidents, cerebral traumas, hemorrhagic or ischemic vascular accidents of the spinal cord, or spinal cord traumas.

3. Use according to one of claims 1 to 2, **characterized in that** G-SCF is combined with at least one other factor intended for local administration or administration by systemic route.

4. Use according to claim 3, **characterized in that** this other factor is selected from at least one of the following factors: BMP (bone morphogenetic protein), FGF (fibroblast growth factor), EGF (epidermal growth factor), IGF (insulin-like growth factor), insulin, KGF (keratinocyte growth factor), TGF (transforming growth factor), interferon, interleukin, VEGF (vascular endothelial growth factor), TNF (tumor necrosis factor), GDNF (glial cell line-derived neurotrophic factor), NGF (neurotrophin nerve growth factor), HGF (hepatocyte growth factor), erythropoietin, PDGF (platelet-derived growth factor), heparan sulfate, prostaglandins, osteoglycin (osteoinductive factor), BCDF (B-cell differentiation factor), GDF-5 (growth and differentiation factor-5), growth hormone; M-CSF (macrophage colony stimulating factor); any known growth factor of human or animal origin, whether extracted or recombinant; or any biological factor strengthening the mobilization capacity of bone marrow stem cells toward the circulating blood.

5. Use according to one of claims 1 to 4, **characterized in that** the G-SCF is human recombinant.

6. Use according to one of claims 1 to 4, **characterized in that** the G-SCF is Filgrastime.

7. Use according to one of claims 1 to 4, **characterized in that** the G-SCF is Lénograstime.

8. Use according to one of claims 1 to 7, **characterized in that** the drug is used with a dosage of G-SCF of 0.1 µg to 1000 µg, preferably of 5 µg to 10 µg, by weight body kilogram and by day.

9. Use according to one of claims 1 to 7, **characterized in that** the drug is used in human medicine.

10. Use according to one of claims 1 to 7, **characterized in that** drug is used in veterinary medicine.

## Patentansprüche

1. Verwendung von G-CSF (Granulozyten-Kolonie stimulierender Faktor) für die Herstellung eines Medikaments zur unterstützenden Behandlung in einem Verfahren der Rekonstruktion von Nervengewebe bei einem Lebewesen, wobei das Medikament zur systemischen Verabreichung bestimmt ist.

2. Verwendung nach Anspruch 1, **dadurch gekennzeichnet, dass** der G-CSF verwendet wird als Medikament bei der Behandlung von ischämischen oder hämorrhagischen Schlaganfällen, zerebralen Traumata, hämorrhagischen oder ischämischen Infarkten des Rückenmarks, Traumata des Rückenmarks.

3. Verwendung nach Anspruch 1 oder 2, **dadurch gekennzeichnet, dass** der G-CSF als Medikament verwendet wird, dass er mit mindestens einem weiteren Faktor kombiniert wird, welcher zur lokalen oder systemischen Verabreichung bestimmt ist.

4. Verwendung nach Anspruch 3, **dadurch gekennzeichnet, dass** dieser weitere Faktor aus mindestens einem der folgenden Faktoren ausgewählt ist: BMP (bone morphogenetic protein), FGF (fibroplast growth factor), EGF (epidermal growth factor), IGF (insulin-like growth factor), Insulin, KGF (keratinocyte growth factor), TGF (transforming growth factor), Interferon, Interleukin, VEGF (vacsular endothelial growth factor), TNF (tumor necrosis factor), GDNF (glial cell line-derived neurotrophic factor), NGF (neurotropin nerve growth factor), HGF (hepatocyte growth factor), Erythropoetin, PDGF (platelet-derived growth factor), Heparansulfat, Prostaglandine, Osteoglycin (osteoinductive factor), BCDF (B cell differentiation factor), GDF-5 (growth and differentiation factor-5), Wachstumsfaktor; M-CSF (macrophage colony stimulating factor); jeder bekannte Wachstumsfaktor menschlichen oder tierischen Ursprungs, extrahiert oder rekombinant; jeder biologische Faktor, der das Mobilisationsvermögen der Stammzellen des Knochenmarks in Richtung des zirkulierenden Blutes verstärkt.

5. Verwendung nach einem der Ansprüche 1 bis 4, **dadurch gekennzeichnet, dass** der G-CSF human rekombinant ist.

6. Verwendung nach einem der Ansprüche 1 bis 4, **dadurch gekennzeichnet, dass** der G-CSF Filgrastim ist.

7. Verwendung nach einem der Ansprüche 1 bis 4, **dadurch gekennzeichnet, dass** der G-CSF Lenograstim ist.

8. Verwendung nach einem der Ansprüche 1 bis 7, **dadurch gekennzeichnet, dass** das Medikament mit einer Dosierung von G-CSF von 0,1 bis 1.000 µg, bevorzugt von 5 bis 10 µg, pro Kilogramm Körpergewicht und pro Tag verwendet wird.

9. Verwendung nach einem der Ansprüche 1 bis 7, **dadurch gekennzeichnet, dass** das Medikament in der Humanmedizin verwendet wird.

10. Verwendung nach einem der Ansprüche 1 bis 7, **dadurch gekennzeichnet, dass** das Medikament in der Veterinärmedizin verwendet wird.
